# EUROPEAN PATENT SPECIFICATION

(11) **EP 2 736 530 B1**
(45) Date of publication and mention of the grant of the patent: **06.06.2018**
(21) Application number: 12817129.5
(22) Date of filing: 27.07.2012
(51) Int. Cl.: A61K 38/20, A61P 7/06

(54) **USE OF IL-12 TO GENERATE ENDOGENOUS ERYTHROPOIETIN**
VERWENDUNG VON IL-12 ZUR ERZEUGUNG VON ENDOGENEM ERYTHROPOETIN
UTILISATION D'IL-12 POUR GÉNÉRER DE L'ÉRYTHROPOÏÉTINE ENDOGÈNE

(30) Priority: 27.07.2011 US 201161512344 P
(43) Date of publication of application: 04.06.2014
(73) Proprietor: Neumedicines, Inc, Pasadena, California 91107 (US)
(72) Inventor: BASILE, Lena, A., Tujunga, CA 91042 (US)
(74) Representative: Herzog, Fiesser & Partner Patentanwälte PartG mbB
(86) International application number: PCT/US2012/048540
(87) International publication number: WO 2013/016634

(56) References cited:
- EP-A1- 1 374 898
- WO-A1-95/24918
- WO-A1-97/16203
- WO-A1-2011/146574
- WO-A2-2005/007093
- US-A- 5 188 828
- US-B2- 7 939 058
- JACKSON ET AL: "Interleukin-12 Enhances Peripheral Hematopoiesis in Vivo", BLOOD, AMERICAN SOCIETY OF HEMATOLOGY, US, vol. 89, no. 9, 1 January 1995 (1995-01-01), pages 2371-2375, XP002993723, ISSN: 0006-4971
- DYBEDAL, I ET AL.: 'IL-12 Directly Enhances In Vitro Murine Erythropoiesis in Combination with IL-4 and Stem Cell Factor.' THE JOURNAL OF IMMUNOLOGY vol. 154, 1995, pages 4950 - 4955, XP055143070
- BASILE ET AL.: 'HemaMaxTM, a Recombinant Human Interleukin-12, Is a Potent Mitigator of Acute Radiation Injury in Mice and Non-Human Primates.' PLOS ONE. vol. 7, no. 2, 23 February 2012, page E30434, XP055143072

## Description

### CROSS REFERENCE TO RELATED APPLICATIONS

This application claims priority to U.S. Provisional Application Serial Number 61/512,344, filed July 27, 2011.

### BACKGROUND

Increasing levels of erythropoietin in a variety of different patients leads to increases in red blood cell production and improved healing as well as protection from damage due to ischemic or hypoxic conditions. An example of a condition to be treated is anemia. Anemia, which is a decrease in the number of circulating red blood cells, is detrimental in patients with a variety of medical conditions to healing of injured tissues and organs. Thus, methods of increasing endogenous erythropoietin and consequently red blood cells and related parameters, such as hemoglobin and hematocrit, is desirable.

Interleukin-12 (IL-12) is a heterodimeric cytokine generally described as a proinflamatory cytokine that regulates the activity of cells involved in the immune response (Fitz et al., J. Exp. Med., 170: 827-45 (1989)). Generally IL-12 stimulates the production of interferon-y (IFN-y) from natural killer (NK) cells and T cells (Lertmemongkolchai et al., J. of Immunology, 166: 1097-105 (2001); Cui et al., Science, 278:1623-6 (1997); Ohteki et al., J. Exp. Med., 189:1981-6 (1999); Airoldi et al., J. of Immunology, 165: 6880-8 (2000)), favors the differentiation of T helper 1 (TH1) cells (Hsieh et al., Science, 260: 547-9 (1993); Manetti et al., J. Exp. Med., 177: 1199-1204 (1993)), and forms a link between innate resistance and adaptive immunity. IL-12 has also been shown to inhibit cancer growth via its immuno-modulatory and anti-angiogenesis effects (Brunda et al., J. Exp. Med., 178: 1223-1230 (1993); Noguchi et al., Proc. Natl. Acad. Sci. U.S.A., 93: 11798-11801 (1996); Giordano et al., J. Exp. Med., 194: 1195-1206 (2001); Colombo et al., Cytokine Growth factor, Rev. 13: 155-168 (2002); Yao et al., Blood, 96: 1900-1905 (2000)). IL-12 is produced mainly by dendritic cells (DC) and phagocytes (macrophages and neutrophils) once they are activated by encountering pathogenic bacteria, fungi or intracellular parasites (Reis et al., J. Exp. Med., 186:1819-1829 (1997); Gazzinelli et al., J. Immunol., 153: 2533-2543 (1994); Dalod et al., J. Exp. Med., 195: 517-528 (2002)). The IL-12 receptor (IL-12 R) has been previously known to be expressed mainly by activated T cells and NK cells (Presky et al., Proc. Natl. Acad. Sci. U.S.A., 93: 14002-14007 (1996); Wu et al., Eur. J. Immunol., 26: 345-50 (1996)).

Generally the production of IL-12 stimulates the production of IFN-γ, which, in turn, enhances the production of IL-12, thus forming a positive feedback loop. Within *in vitro* systems, it has been reported that IL-12 can synergize with other cytokines (IL-3 and SCF for example) to stimulate the proliferation and differentiation of early hematopoietic progenitors (Jacobsen et al., J. Exp. Med., 2: 413-8 (1993); Ploemacher et al., Leukemia, 7: 1381-8 (1993); Hirao et al., Stem Cells, 13: 47-53 (1995)).

IL-12 has been described as being useful in treating hematopoiesis. For example, U.S. Patent No. 7,939,058 for "Uses of IL-12 in hematopoiesis" describes methods for enhancing or stimulating hematopoiesis including administering Interleukin-12 (IL-12) to yield hematopoietic recovery in a mammal in need. The '058 patent describes using IL-12 as an adjuvant therapy to alleviate the hematopoietic toxicities associated with one or more treatment regimens used to combat a disease state. Other described methods are directed to uses of 11-12 for bone marrow preservation or recovery. Other known uses of IL-12 are described in US 2012/0189577 for "Use of IL-12 to increase survival following acute exposure to ionizing radiation"; US 2010-0278778 A1 for "Method for bone marrow preservation or recovery"; US 2011-0206635 and US 2012-0190909, both for "Uses of IL-12 in hematopoiesis"; US 2012-0189577 for "Use of IL-12 to increase survival following acute exposure to ionizing radiation"; WO 2011/146574 for "IL-12 formulations for enhancing hematopoiesis"; WO 2012/050829 for "Uses of IL-12 and the IL-12 receptor positive cell in tissue repair and regeneration"; and PLoS One. 2012;7(2):e30434. Epub 2012 Feb 24 for "HemaMax™, a recombinant human interleukin-12, is a potent mitigator of acute radiation injury in mice and non-human primates".

There remains a need in the art for new compositions and therapies for treating anemia. The present invention satisfies these needs.

### SUMMARY

The present invention relates to recombinant IL-12 for use in increasing the level of endogenous erythropoietin in the blood of a subject according to claim 1. The present disclosure relates to the use of exogenous interleukin-12 (IL-12) for increasing endogenous production of erythropoietin. Other benefits of the present disclosure include tissue repair and regeneration in diseased organs, particularly the kidney, as well as tissues such as skin, muscle, and bone. In the present disclosure, IL-12 is defined as IL-12 or any similar recombinant IL-12 that can provide the therapeutic effect disclosed.

The present disclosure provides methods for increasing endogenous production of erythropoietin in a subject, thereby promoting erythropoiesis, and/or reducing the requirement for red blood cell transfusions, and/or increasing hematocrit and hemoglobin, and/or reducing the requirement for allogeneic blood transfusions, and/or reducing apoptosis, and/or increasing the number of endothelial progenitor cells, and/or improving endothelial cell function, and/or neovascularization, and/or stimulating tissue protection and regenerative pathways, and/or stimulating neurogenesis and/or angiogenesis.

The methods include administering low dose(s) (about 1 to about 1000 ng/kg) of IL-12 to the subject, preferably within a dose range of about 10 ng/kg to about 320 ng/kg, and more preferably between about 16 ng/kg and about 160 ng/kg.

In one aspect, the disclosure provides for a method for generating endogenous erythropoietin in a subject in need of increasing the levels of erythropoietin in the blood comprising administering a therapeutically effective dose of recombinant IL-12 to the subject, wherein the therapeutically effective dose of IL-12 elevates the endogenous erythropoietin blood concentration.

In some embodiments, the subject is human.

In some disclosures, the endogenous erythropoietin level in the blood is increased to between about 40 pg/mL and about 5000 pg/mL. In some disclosures, the endogenous erythropoietin level in the blood is increased to between about 40 pg/ml and about 1000 pg/ml. In some disclosures, the endogenous erythropoietin level in the blood is increased to between about 40 pg/ml and about 500 pg/ml.

In some embodiments, IL-12 is administered in a dose of between about 10 ng/kg body weight and about 320 ng/kg body weight. In some embodiments, IL-12 is administered in a dose of between about 16 ng/kg body weight and about 160 ng/kg body weight. In some embodiments, IL-12 is administered in a dose between about 5 µg and 15 µg.

In some embodiments, IL-12 is administered as a single dose. In some embodiments, IL-12 is administered in multiple doses. In some embodiments, IL-12 is administered biweekly. In some embodiments, IL-12 is administered monthly.

In some disclosures, the erythropoietin levels are increased for at least about 24 hours following IL-12 administration. In some disclosures, the erythropoietin levels are increased for at least about 36 hours, at least about 2 days, at least about 60 hours (2.5 days), at least about 3 days, at least about 84 hours (3.5 days), at least about 4 days, at least about 108 hours (4.5 days), or at least about 5 days following IL-12 administration. In some disclosures, the erythropoietin levels are increased for at least about 10 days following IL-12 administration.

In some embodiments, the recombinant IL-12 is administered to patients with chronic kidney disease before the onset of anemia. In some embodiments, the recombinant IL-12 is administered to patients with chronic kidney disease after the onset of anemia. In some disclosures, the administration of recombinant IL-12 results in improvement in kidney function.

In some disclosures, the recombinant IL-12 is administered to HIV patients with anemia. In some disclosures, the recombinant IL-12 is administered to patients with anemia due to the effect of concomitantly administered chemotherapy. In some disclosures, the recombinant IL-12 is administered to patients with anemia due to the effect of concomitantly administered radiation therapy. In some disclosures, the recombinant IL-12 is administered to patients undergoing surgery.

In some embodiments, the recombinant IL-12 is administered to patients with anemia and cardiovascular disease. In some disclosures, administration of recombinant IL-12 improves neovascularization and heart function in patients with cardiovascular disease.

In some disclosures, the recombinant IL-12 is administered to patients with brain disorders. In some disclosures, the brain disorder is selected from the group comprising stroke, ischemia, traumatic brain injury, traumatic spinal cord injury, epilepsy, Alzheimer's Disease, Parkinson's Disease, amyotrophic lateral sclerosis (ALS), schizophrenia, and multiple sclerosis.

In some disclosures, the recombinant IL-12 results in increased blood levels of erythropoietin for improving neovascularization, wound healing, and tissue protection in non-cardiovascular tissue.

The foregoing general description and following brief description of the drawings and the detailed description are exemplary and explanatory and are intended to provide further explanation of the disclosures and the invention as claimed. Other objects, advantages, and novel features will be readily apparent to those skilled in the art from the following detailed description of the invention.

### BRIEF DESCRIPTION OF THE DRAWINGS

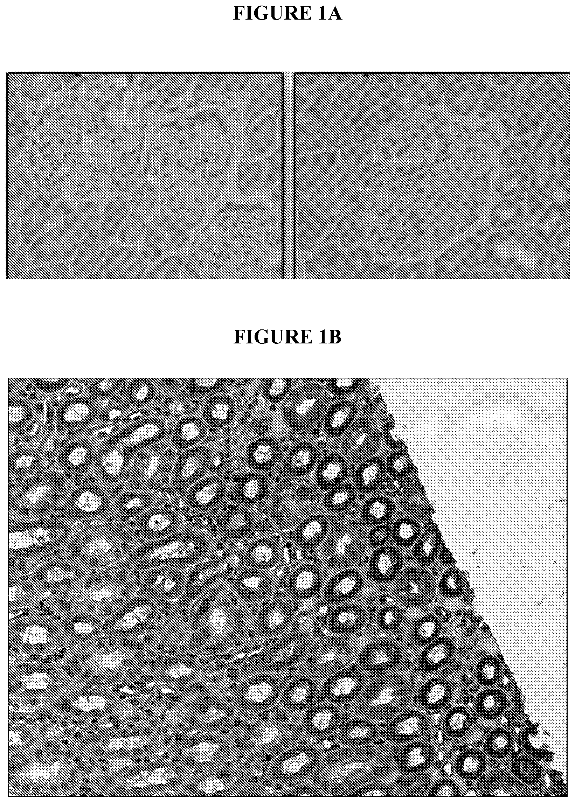

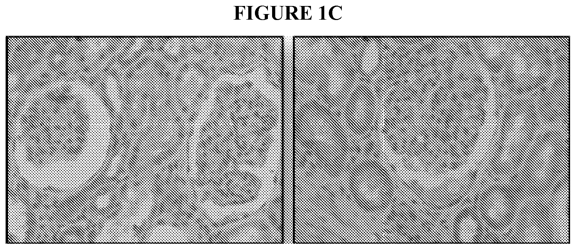

FIG. 1A shows photomicrographs at 400x of medullary tubules in rhesus kidney stained for IL-12Rβ2 expression; FIG. 1B shows a photomicrograph at 20x of rhesus kidney medullary tubules stained for IL-12Rβ2 expression; and FIG. 1C shows photomicrographs at 400x of human kidney medullary tubules stained for IL-12Rβ2. Experimental details are discussed in Example 1.

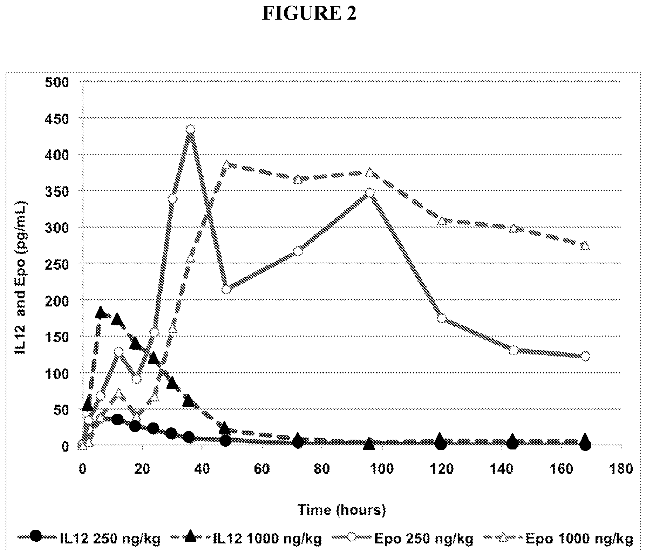

FIG. 2 shows a graph of blood plasma levels of IL-12 (filled circles) and EPO (open circles) in monkeys receiving 250 ng/kg IL-12, as well as blood plasma levels of IL-12 (filled triangles) and EPO (open triangles) in monkeys receiving 1000 ng/kg of IL-12. Experimental details are discussed in Example 2.

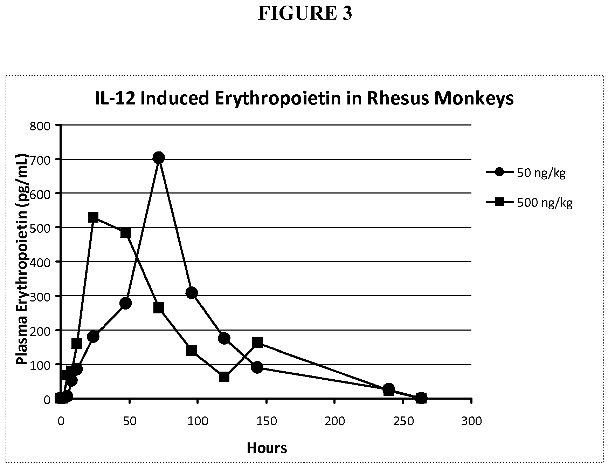

FIG. 3 shows a graph of blood plasma erythropoietin levels over time in rhesus monkeys following administration of 50 and 500 ng/kg of IL-12. Experimental details are discussed in Example 3.

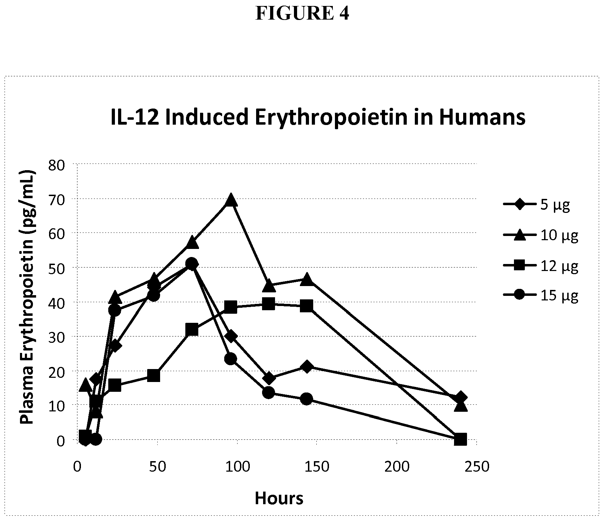

FIG. 4 shows a graph of erythropoietin levels over time in human subjects following administration of 5 µg, 10 µg, 12 µg and 15 µg or vehicle alone via subcutaneous injection. Experimental details are discussed in Example 4.

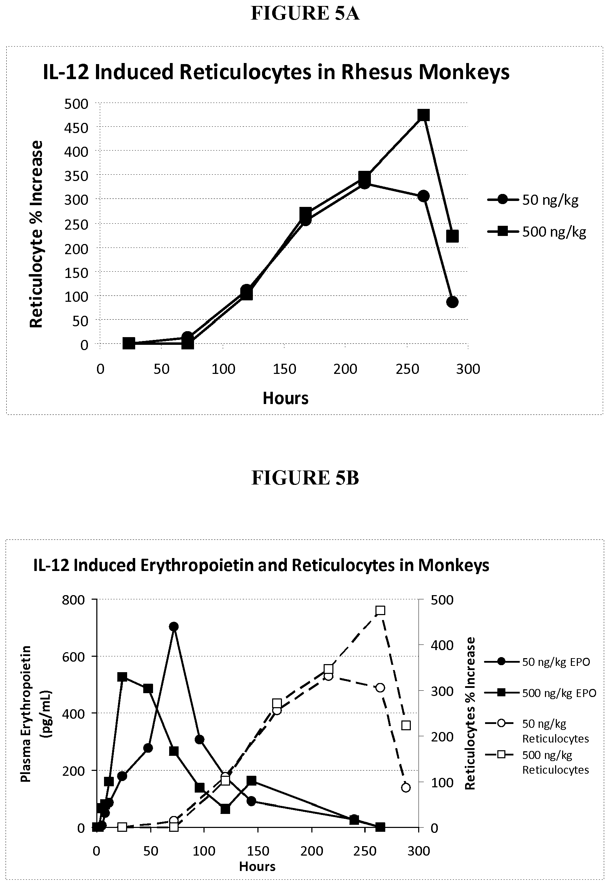

FIG. 5A shows a graph of blood reticulocyte levels following subcutaneous administration of human IL-12 to rhesus monkeys at doses of 50 ng/kg (circles) and 500 ng/kg (squares). FIG. 5B shows a graph of both erythropoietin levels and blood reticulocyte levels following subcutaneous administration of human IL-12 to rhesus monkeys. The closed circles and squares represent data for erythropoietin levels and correlate with the left y-axis. The open circles and squares represent data for reticulocyte levels in the blood and correlate with the right y-axis. Levels presented correspond to reticulocyte percent (%) increases in blood above basal levels and levels observed in vehicle controls.

### DETAILED DESCRIPTION

### I. Definitions

As used herein, the term "about" will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term.

As used herein, except where the context requires otherwise, the term "comprise" and variations of the term, such as "comprising," "comprises" and "comprised" are not intended to exclude other additives, components, integers or steps.

As used herein, "Interleukin-12 (IL-12)" refers to any IL-12 molecule that results in improved cutaneous wound healing, including native IL-12 molecules, variant IL-12 molecules and covalently modified IL-12 molecules, now known or to be developed in the future, produced in any manner known in the art now or to be developed in the future.

Generally, the amino acid sequences of the IL-12 molecule used in embodiments of the invention are derived from the specific mammal to be treated. Thus, for the sake of illustration, for humans, generally human IL-12, or recombinant human IL-12, would be administered to a human in the methods of the invention, and similarly, for felines, for example, the feline IL-12, or recombinant feline IL-12, would be administered to a feline in the methods of the invention. Also included in the invention, however, are certain embodiments where the IL-12 molecule does not derive its amino acid sequence from the mammal that is the subject of the therapeutic methods of the invention. For the sake of illustration, human IL-12 or recombinant human IL-12 may be utilized in a feline mammal.

Still other embodiments of the invention include IL-12 molecules where the native amino acid sequence of IL-12 is altered from the native sequence, but the IL-12 molecule functions to yield the hematopoietic properties of IL-12 that are disclosed herein. Alterations from the native, species-specific amino acid sequence of IL-12 include changes in the primary sequence of IL-12 and encompass deletions and additions to the primary amino acid sequence to yield variant IL-12 molecules. An example of a highly derivatized IL-12 molecule is the redesigned IL-12 molecule produced by Maxygen, Inc. (Leong et al., Proc Natl Acad Sci USA., 100(3): 1163-8 (Feb. 4, 2003)), where the variant IL-12 molecule is produced by a DNA shuffling method. Also included are modified IL-12 molecules in the methods of invention, such as covalent modifications to the IL-12 molecule that increases its shelf life, half-life, potency, solubility, delivery, etc., additions of polyethylene glycol groups, polypropylene glycol, etc., in the manner set forth in U.S. Pat. Nos. 4,640,835; 4,496,689; 4,301,144; 4,670,417; 4,791,192 or 4,179,337. One type of covalent modification of the IL-12 molecule is introduced into the molecule by reacting targeted amino acid residues of the IL-12 polypeptide with an organic derivatizing agent that is capable of reacting with selected side chains or the N- or C-terminal residues of the IL-12 polypeptide. Both native sequence IL-12 and amino acid sequence variants of IL-12 may be covalently modified.

Also as referred to herein, the IL-12 molecule can be produced by various methods known in the art, including recombinant methods. Since it is often difficult to predict in advance the characteristics of a variant IL-12 polypeptide, it will be appreciated that some screening of the recovered variant will be needed to select the optimal variant. A preferred method of assessing a change in the hematological stimulating or enhancing properties of variant IL-12 molecules is via the lethal irradiation rescue protocol disclosed below. Other potential modifications of protein or polypeptide properties such as redox or thermal stability, hydrophobicity, susceptibility to proteolytic degradation, or the tendency to aggregate with carriers or into multimers are assayed by methods well known in the art.

The term "IL-12 receptor" is defined herein as a heterodimeric, membrane-bound receptor for the IL-12 ligand. The IL-12 receptor heterodimer subunits are beta 1 (β1) and beta 2 (β2). In accordance with the present invention, the IL-12 receptor may also bind the IL-12 homodimer and the IL-12 monomer, as defined herein, to form a multimer complex comprising the IL-12 ligand/IL-12 receptor pair and the homodimer and/or the monomer. In the present invention, the multimer complex would further activate the IL-12 ligand/IL-12 receptor pair or may modify the activity of the ligand/receptor pair. In accordance with the present invention, the IL-12 receptor protein is defined to be in its endogenous state as isolated from the IL-12 selected stem cell taken from a donor or a patient. As such, the IL-12 receptor may contain polymorphisms distinct from the canonical amino acid sequence of the β1 and β2 subunits.

The term "One or more therapeutically effective dose(s) of IL-12" refers to any dose administered for any time intervals and for any duration that can improve healing of a cutaneous wound.

The term "therapeutically effective amount or dose" is defined herein as a dose of a substance that produces effects for which it is administered. The exact dose of IL-12 will depend on the purpose of the treatment, the timing of administration of IL-12, certain characteristics of the subject to be treated, and the severity of the cutaneous wound, and is ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

Generally, a dose of a therapeutic agent, according to the methods and compositions of the present invention, can be expressed in terms of the total amount of drug to be administered, *(i.e.,* ng, g, or mg). The dose can be expressed as a weight amount of drug administered to a subject (*e.g.,* 20 ng), or as a ratio of the weight amount of drug per volume unit of carrier (*e.g.,* ng/mL), along with the volume of drug and carrier administered (*e.g.,* 1 mL). Alternatively, the dose can be expressed as a ratio of drug to be administered to weight or surface area of subject receiving the administration (*i.e.,* ng/kg, g/kg, ng,/m², or g/m²). When referring to a dose in terms of the mass to be administered per mass of subject (*i.e.,* ng/kg), it will be understood that doses are not equivalent between different animals, and thus conversion factors will need to be used to ensure that one animal receives the same dose equivalent as another animal. Suitable factors for the conversion of a mouse "dose equivalent" for intraperitoneal (i.p.) injection of IL-12 to a "dose equivalent" of a different animal are given in Table 1 below.

| **Table 1 - Conversion Factors and Equivalent IL-12 Doses for Several Animals** | | | | | |
|---|---|---|---|---|---|
| **Species** | **Weight (kg)** | **Total Dose (ng)** | **Dose (ng/kg)** | **Dose (ng/m²)** | **Conversion Factor** |
| Human | 65 | 25655.82 | 394.7 | 15,000 | 0.0794 |
| Mouse | 0.02 | 99.47 | 4973.44 | 15,000 | 1.0000 |
| Hamster | 0.03 | 130.2 | 4339.87 | 15,000 | 0.8726 |
| Rat | 0.15 | 381.12 | 2540.8 | 15,000 | 0.5109 |

| **Table 1 - Conversion Factors and Equivalent IL-12 Doses for Several Animals** | | | | | |
|---|---|---|---|---|---|
| **Species** | **Weight (kg)** | **Total Dose (ng)** | **Dose (ng/kg)** | **Dose (ng/m²)** | **Conversion Factor** |
| Guinea Pig | 1.00 | 1335 | 1335 | 15,000 | 0.2684 |
| Rabbit | 2.0 | 2381.1 | 1190.65 | 15,000 | 0.2394 |
| Cat | 2.5 | 2956.44 | 1182.57 | 15,000 | 0.2376 |
| Monkey | 3.0 | 3681.75 | 1227.25 | 15,000 | 0.2468 |
| Dog | 8.0 | 6720 | 840 | 15,000 | 0.1689 |

Thus, in one embodiment, doses are given in terms of mass to surface area (*i.e.,* ng/m² or g/m²), which are equivalent for all animals. The following basic conversion factors can be used to convert ng/kg to ng/m²: mouse = 3.0, hamster = 4.1, rat = 6.0, guinea pig = 7.7, human = 38.0 (Cancer Chemother Repts., 50(40):219(1966)).

"Chemotherapy" refers to any therapy that includes natural or synthetic agents now known or to be developed in the medical arts. Examples of chemotherapy include the numerous cancer drugs that are currently available. However, chemotherapy also includes any drug, natural or synthetic, that is intended to treat a disease state. In certain disclosures, chemotherapy may include the administration of several state of the art drugs intended to treat the disease state. Examples include combined chemotherapy with docetaxel, cisplatin, and 5-fluorouracil for patients with locally advanced squamous cell carcinoma of the head (Tsukuda et al., Int. J. Clin. Oncol., 2004 Jun;9(3):161-6), and fludarabine and bendamustine in refractory and relapsed indolent lymphoma (Konigsmann et al., Leuk Lymphoma. 2004;45(9):1821-1827). Another example is the current treatment for HIV infection, or AIDS, currently referred to as HAART, that involves administering at least three antiviral agents to a patient as a treatment for HIV infection. Still another type of chemotherapy are antibiotics and antivirals used to treat pathogenic infections.

"Radiation or radiation therapy or radiation treatment" refers to any therapy where any form of radiation is used to treat the disease state. The instruments that produce the radiation for the radiation therapy are either those instruments currently available or to be available in the future.

"HIV infection" refers to any stage of viral infection or exposure, regardless of the presence of symptoms of HIV infection or AIDS. Further, herein HIV infection refers to the harboring of the HIV virus within cells of a mammal.

### II. Overview

As disclosed herein, it was surprisingly discovered that administration of low doses of recombinant IL-12 to non-human primates and humans results in sustained production of endogenous erythropoietin. Increased and sustained production of erythropoietin has been shown to be beneficial in patients that suffer from a variety of conditions, including chronic renal failure, HIV patients, cancer patients being treated with chemotherapy and/or radiation therapy, patients with cardiovascular disease and/or congestive heart failure, patients with tissue injury, as well as patients suffering from neurological conditions.

### A. Erythropoietin in Chronic Kidney Disease and Chronic Renal Failure

Endogenous production of erythropoietin is normally regulated by the level of tissue oxygenation. Hypoxia and anemia generally increase the production of erythropoietin, which in turn stimulates erythropoiesis. In normal subjects, plasma erythropoietin levels range from 0.01 to 0.03 Units/mL and increase up to 100- to 1000-fold during hypoxia or anemia (1 mUnits/ml = 10 pg/ml). In contrast, in patients with chronic renal failure (CRF), production of erythropoietin is impaired, and this erythropoietin deficiency is the primary cause of their anemia.

Chronic renal failure is the clinical situation in which there is a progressive and usually irreversible decline in kidney function. Such patients may manifest the sequelae of renal dysfunction, including anemia, but do not necessarily require regular dialysis. Patients with end-stage renal disease (ESRD) are those patients with CRF who require regular dialysis or kidney transplantation for survival. Administration of exogenous erythropoietin stimulates erythropoiesis in anemic patients with CRF, including both patients on dialysis and those who do not require regular dialysis.

Chronic Kidney Disease (CKD; also known as Chronic Renal Disease) is a progressive loss of renal function. CKD is often diagnosed by increases in blood levels of creatinine, although early stage CKD may be diagnosed by detection of proteins and cells in urine. CKD severity is broken up into five stages based on estimated glomerular filtration rate, with stage 1 being the least severe and stage 5 correlating with chronic renal failure. As kidney function declines in CKD, anemia is a common symptom.

In some embodiments of the present invention, a therapeutically effective amount of IL-12 is administered to mammals, such as humans, with early stage CKD that do not show signs of anemia (e.g. stage 1 or 2). In some embodiments, a therapeutically effective amount of IL-12 is administered to mammals, such as humans, with later stage CKD with anemia (*e.g.* stages 3-5). Administration of IL-12 to increase levels of erythropoietin in patients with CKD will slow or halt the progression of CKD.

### B. Erythropoietin in HIV

Stimulation of erythropoiesis using exogenous erythropoietin also reduces the requirement for red blood cell transfusions in a variety of patients. Administration of erythropoietin is known to increase hematocrit in HIV-infected patients with anemia related to therapy with zidovudine.

In some disclosures, a therapeutically effective amount of IL-12 is administered to a patient being treated for HIV and having anemia due to treatment, such as zidovudine.

### C. Erythropoietin in Blood Transfusions

Erythropoietin is also know to reduce the requirement for red blood cell transfusions in cancer patients receiving chemotherapy and/or radiation therapy and in anemic patients who are at high risk for perioperative blood loss from elective, noncardiac, nonvascular surgery.

In some disclosures, a therapeutically effective amount of IL-12 is administered to a patient receiving chemotherapy and/or radiation therapy. In some disclosures, a therapeutically effective amount of IL-12 is administered to a patient at high risk for perioperative blood loss from elective, noncardiac, nonvascular surgery.

### D. Erythropoietin and anemia in cardiovascular disease

In both controlled and uncontrolled studies of congestive heart failure, the correction of the anemia with erythropoietin and oral or intravenous (IV) iron has been associated with improvement in many cardiac and renal parameters and an increased quality of life (QoL). Anemia may also play a role in the worsening of acute myocardial infarction and chronic coronary heart disease (CHD) and in the cardiovascular complications of renal transplantation. Anemia, congestive heart failure (CHF) and chronic kidney disease (CKD) interact as a vicious circle so as to cause or worsen each other, the so-called cardio renal anemia syndrome. Only adequate treatment of all three conditions can prevent the CHF and CKD from progressing. Siverberg et al., Int. Urol. Nephrol., 38(2):295-310 (2006).

Erythropoietin improves heart function by reducing apoptosis of cardiac and endothelial cells, increasing the number of endothelial progenitor cells, and improving endothelial cell function and neovascularization of the heart. Raddino et al., Monaldi Arch. Chest Dis., 70(4):206-13 (2008).

In some embodiments of the invention, a therapeutically effective amount of IL-12 is administered to patients with CHD, CHF, and/or cardiovascular disease, or patients that have received a renal transplant to increase levels of erythropoietin.

### E. Erythropoietin in tissue protection and repair (non-cardiovascular)

Erythropoietin acts as a locally produced antagonist of proinflammatory cytokines that are generated by the innate immune response in response to infection, trauma, or metabolic stress. Specifically, erythropoietin inhibits apoptosis of cells surrounding a locus of injury, reduces the influx of inflammatory cells, and recruits tissue-specific stem cells and endothelial progenitor cells. Available evidence suggests that these multiple, non-erythropoietic effects of erythropoietin are mediated by a tissue protective receptor (TPR) may be distinct from the homodimeric receptor responsible for erythropoiesis, resulting in tissue protection, repair and regeneration of damaged tissue.. Hand et al., JInvestig. Med., 59(7):1073-82 (2011).

In some disclosures, a therapeutically effective amount of IL-12 is administered to patients with non-cardiovascular tissue damage to increase levels of erythropoietin.

### F. Erythropoietin and tissue protection and repair in neurological disease and injury

Erythropoietin has been shown to be neurotrophic and neuroprotective *in vitro* and in animal models of neuronal injury, as well as in clinical trials for certain neurological diseases and disorders. Erythropoietin and the erythropoietin receptor are expressed by neurons, glial cells and cerebrovascular endothelium and is greatly increased during hypoxia and metabolic stress. Administration of erythropoietin and its chemical variants have been shown to have beneficial effects in animal models of stroke and ischemia, traumatic brain and spinal cord injury, epilepsy, as well as neurodegenerative diseases such as Alzheimer's Disease, Parkinson's Disease, and amyotrophic lateral sclerosis (ALS). Siren et al, Neurotherapeutics, 6(1):108-27 (Jan. 2009); Yang et al. Acta Neurobiol Exp (Wars), 67(2): 141-8 (2007). The beneficial effects of erythropoietin have also been demonstrated in clinical studies of stroke, schizophrenia, and multiple sclerosis. Siren et al, Neurotherapeutics, 6(1):108-27 (Jan. 2009). Erythropoietin protects neurons both directly, by preventing apoptosis, and indirectly, by modulating inflammatory processes and stimulating neurogenesis and angiogenesis.

In some disclosures, a therapeutically effective amount of IL-12 is administered to patients with brain disorders, including stroke, ischemia, traumatic brain injury, traumatic spinal cord injury, epilepsy, Alzheimer's Disease, Parkinson's Disease, amyotrophic lateral sclerosis (ALS), schizophrenia, and/or multiple sclerosis. In some disclosures, administration of low doses of IL-12 result in reduced lesion size, reduction or halting of neuronal loss and/or brain atrophy, and improved cognition and motor function.

### III. IL-12 Dosing and Dosages

Generally the IL-12 doses used for treating patients will be high enough to be effective for the treatment of anemia, but low enough to mitigate negative side effects associated with IL-12 administrations, including for example, radiosensitivity of the GI tract (associated with radiation exposure) and IFN-γ up-regulation.

In one aspect, a single dose of IL-12 is sufficient to confer increased endogenous erythropoietin in a subject. In other aspects, IL-12 may be administered in more than one dose, such as about 2, about 3, about 4, about 5 or more doses. IL-12 doses can be given daily or over any desired time period, such as once or more every other day, every 2 days, every 3 days, every 4 days, every 5 days, every 6 days, or every 7 days. IL-12 doses can also be given once or more every week, every other week, every 3 weeks every 4 weeks, every month, every other month, etc.

Accordingly, in one aspect, the present invention provides recombinant IL-12 for use in increasing the level of endogenous erythropoietin in the blood of a subject according to claim 1 comprising the administration of one or more doses of IL-12 to a subject. In one embodiment, the dose of IL-12 is less than about 100 µg/m². In another embodiment, the dose of IL-12 is less than about 75 µg/m², or less than about 400 ng/kg (15 µg/m²). In another embodiment, the dose can be between about 1 µg/m² and about 100 µg/m². Other exemplary IL-12 dosages include less than about 1 µg/m² or about 1 µg/m², less than about 3 µg/m² or about 3 µg/m², less than about 4 µg/m² or about 4 µg/m², less than about 5 µg/m² or about 5 µg/m², less than about 6 µg/m² or about 6 µg/m², less than about 7 µg/m² or about 7 µg/m², less than about 8 µg/m² or about 8 µg/m², less than about 9 µg/m² or about 9 µg/m², less than about 10 µg/m² or about 10 µg/m², less than about 11 µg/m² or about 11 µg/m², less than about 12 µg/m² or about 12 µg/m², less than about 15 µg/m² or about 15 µg/m², less than about 20 µg/m² or about 20 µg/m², less than about 25 µg/m² or about 25 µg/m², less than about 30 µg/m² or about 30 µg/m², less than about 35 µg/m² or about 35 µg/m², less than about 40 µg/m² or about 40 µg/m², less than about 45 µg/m² or about 45 µg/m², less than about 50 µg/m² or about 50 µg/m², less than about 55 µg/m² or about 55 µg/m², less than about 60 µg/m² or about 60 µg/m², less than about 65 µg/m² or about 65 µg/m², less than about 70 µg/m² or about 70 µg/m², less than about 75 µg/m² or about 75 µg/m², less than about 80 µg/m² or about 80 µg/m², less than about 85 µg/m² or about 85 µg/m², less than about 90 µg/m² or about 90 µg/m², less than about 95 µg/m² or about 95 µg/m², less than about 100 µg/m² or about 100 µg/m², less than about 900 ng/m² or about 900 ng/m², less than about 800 ng/m² or about 800 ng/m², less than about 700 ng/m² or about 700 ng/m², less than about 600 ng/m² or about 600 ng/m², less than about 500 ng/m² or about 500 ng/m², less than about 400 ng/m² or about 400 ng/m², less than about 300 ng/m² or about 300 ng/m², less than about 250 ng/m² or about 250 ng/m², less than about 200 ng/m² or about 200 ng/m², less than about 100 ng/m² or about 100 ng/m², and all doses in-between.

In one embodiment of the invention, the dosage of IL-12 is between about 1 ng/mL and about 10 µg/mL. In another embodiment, the dosage of IL-12 is between about 10 ng/mL and about 5 µg/mL. In other embodiments of the invention, the dosage of IL-12 is about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, about 250, about 260, about 270, about 280, about 290, about 300, about 310, about 320, about 330, about 340, about 350, about 360, about 370, about 380, about 390, or about 400 ng/mL.

In one embodiment of the invention, the dosage of IL-12 is between about 10 ng/kg and about 1000 ng/kg. In some embodiments of the invention, the dosage of IL-12 is between about 10 ng/kg and 500 ng/kg. In some embodiments of the invention, the dosage of IL-12 is between about 16 ng/kg and 320 ng/kg. In other embodiments of the invention, the dosage of IL-12 is about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, about 250, about 260, about 270, about 280, about 290, about 300, about 310, about 320, ng/kg.

When administered in multiple doses, *i.e.* two, three, four, or more, the first IL-12 dose and subsequent IL-12 dose(s) can be equivalent doses, or they can be different dose amounts. For example, in certain embodiments, subsequent dose(s) can be administered at about 90% of the initial dose, or at about 80%, about 75%, about 70%, about 60%, about 50%, about 40%, about 30%, about 25%, about 20%, or about 10% or less of the original dose.

As described in Example 4 below, IL-12 administered to healthy humans at doses of 5, 10, 12, and 15 µg resulted in an increase of erythropoietin which increased and ebbed over time in each individual. Induced EPO levels peaked over a range of 40 pg/mL to 70 pg/mL for the four dose groups. The time to maximum blood plasma level for the four groups ranged from 72 - 120 hours. FIG. 3 shows a graph of induced erythropoietin levels over time in human healthy volunteers following administration of the various dosages of IL-12. Table 2 below summarizes the pharmacokinetic parameters for EPO at both doses.

| **Table 2: Pharmacokinetic Parameters of EPO following IL-12 Administration in Humans** | | | | |
|---|---|---|---|---|
| **IL-12-induced EPO** | **Tₘₐₓ (hours)** | **T_{1/2} (hours)** | **Cₘₐₓ (pg/mL)** | **AUC_{∞} (pg.hour/mL)** |
| 5 µg | 72 | 120 | 51 | 6150 |
| 10 µg | 96 | 120 | 69 | 9450 |
| 12 µg | 120 | 117 | 39 | 5760 |
| 15 µg | 72 | 114 | 51 | 5480 |

Based on this data, larger doses of IL-12 do not necessarily correlate with an increase in Cₘₐₓ or in AUC. Specifically, as shown in Table 2, does of 5 and 15 µg *both* resulted in a Cₘₐₓ of 51 pc/mL. Similarly, a dose of 10 µg resulted in a significantly higher AUC as compared to doses and 15 µg: 9450 pg.hour/mL AUC for the 10 µg dose, as compared to 5760 pg.hour/mL and 5480 pg.hour/mL AUC for the 12 and 15 µg doses, respectively. All human doses presented and evaluated as described above produced large increases in endogenous erythropoietin due to IL-12 administration over similar time courses. There is a broad effective dose range for IL-12 induction of endogenous erythropoietin in human beings.

### IV. IL-12 Compositions

For general descriptions relating IL-12, see U.S. Pat. Nos. 5,573,764, 5,648,072, 5,648,467, 5,744,132, 5,756,085, 5,853,714 and 6,683,046.

In certain embodiments, the IL-12 is a recombinant mammalian IL-12, murine IL-12 (mIL-12), recombinant murine IL-12 (rmIL-12), human IL-12 (hIL-12), recombinant human IL-12 (rhIL-12), canine rIL-12, feline rIL-12, bovine rIL-I2, equine rIL-I2, or biologically active variants or fragments thereof. In one specific embodiment, the rhlL-I2 is HemaMax™ (Neumedicines Inc.). In certain embodiments, the IL-12 can be modified so as to reduce the immunogenicity of the protein after administration to a subject. Methods of reducing the immunogenicity of a protein are well known in the art and include, for example, modifying the protein with one or water soluble polymers, such as a PEG, a PEO, a carbohydrate, a polysialic acid, and the like.

It is well known that solutions of proteins that are formulated at low concentrations are susceptible to loss of a significant fraction of the protein prior to administration. One major cause of this problem is adsorption of the protein on the sides of tubes, vials, syringes, and the like. Accordingly, in certain aspects, when administered at low or ultralow doses, it will be beneficial to administer IL-12 along with a suitable carrier molecule or bulking agent. In one embodiment, the carrier agent may be a protein suitable for pharmaceutical administration, such as albumin. Generally, the carrier molecule or protein will be present in the formulation in excess of IL-12 to minimize the amount of IL-12 lost prior to administration. In certain embodiments, the carrier will be present at a concentration of at least about 2 times the concentration of IL-12, or at a concentration of at least about 3, at least about 4, at least about 5, at least about 6, at least about 7, at least about 8, at least about 9, at least about 10, at least about 25, at least about 50, at least about 100, or more times the concentration of IL-12 in the formulation.

IL-12 compositions provided herein and used according to the invention can be formulated for administration via any known method, but preferably orally, topically, subcutaneously, or intramuscularly. Further, an efficacious dose of IL-12 may differ with different routes of administration.

In some embodiments, the formulations provided herein further comprise one or more pharmaceutically acceptable excipients, carriers, and/or diluents. In addition, the formulations provided herein may further comprise other medicinal agents, carriers, adjuvants, diluents, tissue permeation enhancers, solubilizers, and the like. Methods for preparing compositions and formulations for pharmaceutical administration are known to those skilled in the art (see, for example, Remington's Pharmaceutical Sciences, 18TH ED., Mack Publishing Co., Easton, PA (1990)). Formulations used according to the methods of the invention may include, for example, those taught in U.S. Patent No. 5,744,132.

### EXAMPLES

### Example 1: Expression of IL-12Rβ2 in Primate Kidney

Expression of IL-12Rβ2 in kidney tissue of rhesus monkeys and humans was examined.

### Materials and Methods

Paraffin-embedded and sectioned tissues from rhesus monkey and human kidney were supplied by Cytopathology Diagnostics Center, Inc (Duarte, CA) and Biomax, Inc.. Sections were deparaffinized with xylene and re-hydrated with ethanol (100% (2x), 95% (2x), 70% (1x), 3 minutes each and H₂O for 5 minutes) and subjected to heat-induced epitope retrieval (HIER) by microwaving in a pressure cooker for 10 minutes at 700W in citrate buffer, pH 6. Endogenous peroxidase was blocked by incubation with 0.3% H₂O₂ (VWR; San Francisco, CA) for 30 minutes at room temperature. Sections were washed in PBS/0.2% Tween® 20 and blocked using Background Sniper (Biocare Medical, LLC; Concord, CA) for 15 minutes followed by incubation with rabbit antibody to IL-12Rβ2 (Sigma; St Louis, Missouri) diluted 1:25 in primary antibody diluent (Diagnostic Biosystems; Pleasanton, CA) for 2 hours. The sections were washed and incubated with peroxidase conjugated anti-Rabbit IgG (ImmPRESS; Vector Laboratories; Burlingame, CA) for 30 minutes followed by washing with tap water. Peroxidase labeled cells are visualized by incubation in AEC substrate (ImmPACT AEC; Vector Laboratories). Tissue was counterstained with Churukian-Allison-Tacha (CAT) Hematoxylin (Biocare Medical) followed by washing with tap water. Sections were immersed in Vectamount™ (Vector Laboratories), covered with a cover slip, and sealed with clear nail polish. Slides were visualized using an Olympus BX41 compound microscope. Images were acquired using Infinity Analyze software v.6.0.

### Results

Kidney tissue from rhesus monkeys and humans stained strongly in the medullary tubules for IL-12Rβ2. Cells stained for IL-12Rβ2 were epithelial or endothelial in origin. Some of the stained cells could also be mesangial (cytokine-secreting cells). Staining for IL-12Rβ2 expression was not seen in human cortical kidney (data not shown). FIG. 1A shows a photomicrograph at 400x of medullary tubules in rhesus kidney stained for IL-12Rβ2 expression; FIG. 1B shows a photomicrograph at 20x of rhesus kidney medullary tubules stained for IL-12Rβ2 expression; FIG. 1C shows a photomicrograph at 400x of medullary tubules in human kidney. IL-12Rβ2 expression in medullary tubules normally responsible for electrolyte balance and water reabsorption is significant in that the primary location of expression of erythropoietin has been shown to arise from kidney tubules. Tubule expression in rhesus monkeys is consistent with that observed for human tissue stained for IL-12Rβ2 expression (see, for example, the Human Protein Atlas on the worldwide web).

### Example 2: IL-12 Induction of Erythropoietin in Rhesus Monkeys at Doses of 250 ng/kg and 1000 ng/kg

Two different doses of recombinant human IL-12 were administered to rhesus monkeys and erythropoietin levels in blood samples from the test subjects was measured.

### Materials and Methods

Recombinant IL-12 was administered to six healthy rhesus monkeys at a single dose of either 250 ng/kg or 1000 ng/kg (three subjects per dose) subcutaneously to the intrascapular area. The levels of both IL-12 and erythropoietin (EPO) were measured in blood samples from the time IL-12 was administered until more than 160 hours following administration. Blood samples were be collected by venipuncture into tubes containing K2-EDTA as anticoagulant and kept on wet ice pending centrifugation (maximum 30 minutes). Samples were centrifuged under refrigeration (approximately +4°C at 1500 g RCF) for 10 minutes. Plasma was aliquoted at 200 µL/tube, placed on dry ice pending storage at approximately -70°C until transferred to assay laboratory. Analyses of human IL-12 and rhesus erythropoietin in blood plasma were done using two commercially available validated ELISA assays, which separately recognized human IL-12 and rhesus erythropoietin (Human IL-12 (p70) ELISA Max Deluxe, BioLegend, # 431704; Human EPO ELISA Quantikine, R&D Systems # DEP00)

### Results

Recombinant human IL-12 at two different doses (250 ng/kg and 1000 ng/kg) induces endogenous erythropoietin production in healthy, normal rhesus monkeys. FIG. 2 shows a graph of blood levels of IL-12 (filled circles) and EPO (open circles) in monkeys receiving 250 ng/kg IL-12, as well as blood levels of IL-12 (filled triangles) and EPO (open triangles) in monkeys receiving 1000 ng/kg of IL-12. The 250 ng/kg dose and 1000 ng/kg dose increased the peak level of endogenous erythropoietin to 434.2 pg/mL and 386.1 pg/mL, respectively. The 250 ng/kg and 100 ng/kg doses in monkeys are equivalent to human doses of about 80 ng/kg and 320 ng/kg, respectively. Table 3 below summarizes the pharmacokinetic parameters for EPO at both doses.

| **Table 3: Pharmacokinetic Parameters of EPO following IL-12 Administration in Rhesus Monkeys** | | | | |
|---|---|---|---|---|
| **IL-12-induced EPO** | **Tₘₐₓ (hours)** | **T_{1/2} (hours)** | **Cₘₐₓ (pg/mL)** | **AUC_{∞} (pg.hour/mL)** |
| 250 ng/kg | 36 | 86 | 434 | 51167 |
| 1000 ng/kg | 48 | 233 | 386 | 139403 |

### Example 3: IL-12 Induction of Erythropoietin in Rhesus Monkeys at Doses between 50 ng/kg and 500 ng/kg

The purpose of this example was to demonstrate the induction of erythropoietin in Rhesus monkeys following administration of IL-12.

Rhesus monkeys (18 subjects) were administered recombinant human IL-12 or vehicle control (N=3 or 4) one time via subcutaneous injection in the intrascapular area. The doses of IL-12 administered were 50, 100, 250, and 500 ng/kg. Levels of erythropoietin (EPO) were measured in blood plasma samples from the time of IL-12 administration to 264 hours (11 days) following IL-12 administration. IL-12 induced erythropoietin levels were determined and analyzed. Blood samples were be collected by venipuncture into tubes containing K2-EDTA as anticoagulant and kept on wet ice pending centrifugation (maximum 30 minutes). Samples were centrifuged under refrigeration (approximately +4°C at 1500 g RCF) for 10 minutes. Plasma was aliquoted at 200 µL/tube, placed on dry ice pending storage at approximately -70°C until transfered to assay laboratory. Analysis of rhesus erythropoietin in blood plasma was done using a commercially available validated ELISA assay which recognized rhesus erythropoietin (Human EPO ELISA Quantikine; R&D Systems # DEP00).

Recombinant human IL-12 over a ten-fold dose range (50 ng/kg to 500 ng/kg) induces endogenous erythropoietin production in healthy, normal rhesus monkeys. FIG. 2 shows a graph of blood plasma levels of EPO in monkeys receiving 50 ng/kg IL-12 (circles), as well as in monkeys receiving 500 ng/kg of IL-12 (squares). The 50 ng/kg dose and 500 ng/kg dose increased the peak level of endogenous erythropoietin to 701 pg/mL and 527 pg/mL, respectively. The 50 ng/kg and 500 ng/kg doses in monkeys are equivalent to human doses of about 16 ng/kg (1.12 µg in an average person) and 160 ng/kg (11.2 µg in an average person) respectively. Table 4 below summarizes the pharmacokinetic parameters for EPO at the 50 ng/kg and 500 ng/kgdoses. Similar results were found for intermediate IL-12 doses of 100 ng/kg and 250 ng/kg.

| **Table 4: Pharmacokinetic Parameters of EPO following IL-12 Administration in Rhesus Monkeys** | | | | |
|---|---|---|---|---|
| **IL-12-induced EPO** | **Tₘₐₓ (hours)** | **T_{1/2} (hours)** | **Cₘₐₓ (pg/mL)** | **AUC_{∞} (pg.hour/mL)** |
| 50 ng/kg | 72 | 130 | 701 | 59585 |
| 500 ng/kg | 24 | 130 | 527 | 48325 |

### Example 4: IL-12 Induction of Erythropoietin in Humans at Doses between 5 µg and 15 µg

The purpose of this example was to demonstrate the induction of erythropoietin in humans following administration of IL-12.

In a human clinical trial in healthy human volunteers, participants were administered human IL-12 at doses of 5 µg, 10 µg, 12 µg and 15 µg or vehicle/placebo alone via subcutaneous injection in the abdomen. Levels of blood plasma erythropoietin were measured from the time of IL-12 or vehicle administration to 240 hours (10 days) following IL-12 or vehicle administration. 16 participants received IL-12 (four participants per dose group) and 16 participants received vehicle/placebo alone. IL-12-induced erythropoietin levels over and above basal levels and vehicle control levels were determined and analyzed. Blood samples were be collected by venipuncture into tubes containing K2-EDTA as anticoagulant and kept on wet ice pending centrifugation (maximum 30 minutes). Samples were centrifuged under refrigeration (approximately +4°C at 1500 g RCF) for 10 minutes. Plasma was aliquoted at 200 µL/tube, placed on dry ice pending storage at approximately - 70°C until transfered to assay laboratory. Analysis of human erythropoietin in blood plasma was done using commercially available validated ELISA assay which recognized human erythropoietin (Human EPO ELISA Quantikine; R&D Systems # DEP00). Reticulocytes were counted using a blood analyzer (ADVIA 120 Analyzer).

Administration of IL-12 caused an increase of erythropoietin which increased and ebbed over time in each individual. Induced EPO levels peaked over a range of 40 pg/mL to 70 pg/mL for the four dose groups. The time to maximum blood plasma level for the four groups ranged from 72 - 120 hours. FIG. 3 shows a graph of induced erythropoietin levels over time in human healthy volunteers following administration of the various dosages of IL-12. Table 5 below summarizes the pharmacokinetic parameters for EPO at both doses.

| **Table 5: Pharmacokinetic Parameters of EPO following IL-12 Administration in Humans** | | | | |
|---|---|---|---|---|
| **IL-12-induced EPO** | **Tₘₐₓ (hours)** | **T_{1/2} (hours)** | **Cₘₐₓ (pg/mL)** | **AUC_{∞} (pg.hour/mL)** |
| 5 µg | 72 | 120 | 51 | 6150 |
| 10 µg | 96 | 120 | 69 | 9450 |
| 12 µg | 120 | 117 | 39 | 5760 |
| 15 µg | 72 | 114 | 51 | 5480 |

### Example 5: IL-12 Induction of Reticulocyte in Rhesus Monkeys at Doses of 50 ng/kg to 500 ng/kg

Four different doses of recombinant human IL-12 was administered to rhesus monkeys and reticulocyte levels in blood samples from the test subjects were measured.

### Materials and Methods

Recombinant IL-12 was administered one time via subcutaneous injection in the intrascapular area to 18 healthy rhesus monkeys at a single dose of either vehicle, 50 ng/kg, 100 ng/kg, 250 ng/kg, or 500 ng/kg. The levels reticulocytes were measured in blood samples from the time IL-12 was administered until 288 hours (12 days) following administration. IL-12 induced reticulocyte levels were determined and analyzed.

### Results

Recombinant human IL-12 over a ten-fold dose range (50 ng/kg and 500 ng/kg) induces generation of reticulocytes in healthy, normal rhesus monkeys. Reticulocytes are precursor cells to red blood cells. FIG. 5A shows a graph of blood reticulocyte levels in monkeys receiving, for example, 50 ng/kg IL-12 (circles), as well as in monkeys receiving 500 ng/kg of IL-12 (squares). Reticulocyte levels are presented as percent increase above basal and vehicle levels and thus reflect the increase in reticulocytes induced by the single subcutaneous injection of IL-12. The 50 ng/kg dose and 500 ng/kg dose increased the level of reticulocytes compared to basal levels and vehicle levels. The increase in reticulocyte levels began at about 72 hours post-injection and began decreasing at about 288 hours (12 days) following IL-12 injection. FIG. 5B is a graph of both erythropoietin levels (see Example 3 and FIG. 3) and reticulocyte levels following a single injection of IL-12.

Peak increases of reticulocytes of approximately 330% (3.3 fold) and 470% (4.7 fold) occurred in the 50 ng/kg and 500 ng/kg doses, respectively. Peak times of increase were 216 hours (9 days) and 264 hours (11 days) for the 50 ng/kg dose and 500 ng/kg dose respectively. The reticulocyte generation induced by IL-12 began to be measurable in the blood three days after administration of IL-12. The 50 ng/kg and 500 ng/kg doses in monkeys are equivalent to human doses of about 16 ng/kg and 160 ng/kg, respectively which respectively correspond to a total dose of 1.12 µg and 11.2 µg in an average sized person.

| **Table 6: Pharmacokinetic Parameters of Reticulocyte Generation Following IL-12 Administration in Rhesus Monkeys** | | | |
|---|---|---|---|
| **IL-12-induced Reticulocytes** | **Tₘₐₓ (hours)** | **T_{1/2} (hours)** | **%Increaseₘₐₓ** |
| 50 ng/kg | 216 | 114 | 330 |
| 500 ng/kg | 264 | 114 | 470 |

The above examples are given to illustrate the present invention. It should be understood, however, that the scope of the invention is not to be limited to the specific conditions or details described in these examples.

It will be apparent to those skilled in the art that various modifications and variations can be made in the compositions of the present invention. Thus, it is intended that the present invention cover the modifications and variations of this invention provided they come within the scope of the appended claims.

## Claims

1. Recombinant IL-12 for use in increasing the level of endogenous erythropoietin in the blood of a subject that has chronic renal failure, chronic kidney disease, cardiovascular disease, or congestive heart failure.

2. The recombinant IL-12 for use of claim 1, wherein the subject is human.

3. The recombinant IL-12 for use of claim 2, wherein recombinant IL-12 is administered to the subject in a dose of:
(a) between about 5 ng/kg body weight and about 500 ng/kg body weight;
(b) between about 10 ng/kg body weight and about 320 ng/kg body weight;
(c) between about 16 ng/kg body weight and about 160 ng/kg body weight; or
(d) between about 5 µg and 15 µg.

4. The recombinant IL-12 for use of any one of claims 1-3, wherein recombinant IL-12 is administered to the subject as a single dose.

5. The recombinant IL-12 for use of any one of claims 1-3, wherein recombinant IL-12 is administered to the subject in multiple doses.

6. The recombinant IL-12 for use of any one of claims 1-3 and 5, wherein recombinant IL-12 is:
(a) administered to the subject once a day, once every two days, once every 3 days, once every 4 days, once every 5 days, once every 6 days, once every week, once every other week, once every 3 weeks, or once every 4 weeks; or
(b) administered to the subject monthly.

7. The recombinant IL-12 for use of any one of claims 1-6, wherein the subject has:
(a) chronic kidney disease prior to the onset of anemia; or
(b) chronic kidney disease after the onset of anemia.

8. The recombinant IL-12 for use of any one of claims 1-6, wherein the subject has anemia and cardiovascular disease.

## Patentansprüche

1. Rekombinantes IL-12 zur Verwendung beim Erhöhen des Spiegels von endogenem Erythropoietin im Blut eines Individuums, bei dem chronisches Nierenversagen, chronische Nierenkrankheit, Herz-Kreislauf-Erkrankung oder kongestive Herzinsuffizienz vorliegt.

2. Rekombinantes IL-12 zur Verwendung nach Anspruch 1, wobei es sich bei dem Individuum um einen Menschen handelt.

3. Rekombinantes IL-12 zur Verwendung nach Anspruch 2, wobei dem Individuum rekombinantes IL-12 in einer Dosis von
(a) zwischen etwa 5 ng/kg Körpergewicht und etwa 500 ng/kg Körpergewicht;
(b) zwischen etwa 10 ng/kg Körpergewicht und etwa 320 ng/kg Körpergewicht;
(c) zwischen etwa 16 ng/kg Körpergewicht und etwa 160 ng/kg Körpergewicht; oder
(d) zwischen etwa 5 µg und 15 µg
verabreicht wird.

4. Rekombinantes IL-12 zur Verwendung nach einem der Ansprüche 1-3, wobei dem Individuum rekombinantes IL-12 als Einzeldosis verabreicht wird.

5. Rekombinantes IL-12 zur Verwendung nach einem der Ansprüche 1-3, wobei dem Individuum rekombinantes IL-12 in mehreren Dosen verabreicht wird.

6. Rekombinantes IL-12 zur Verwendung nach einem der Ansprüche 1-3 und 5, wobei rekombinantes IL-12
(a) dem Individuum einmal am Tag, einmal alle zwei Tage, einmal alle 3 Tage, einmal alle 4 Tage, einmal alle 5 Tage, einmal alle 6 Tage, einmal pro Woche, einmal alle zwei Wochen, einmal alle 3 Wochen oder einmal alle 4 Wochen verabreicht wird; oder
(b) dem Individuum monatlich verabreicht wird.

7. Rekombinantes IL-12 zur Verwendung nach einem der Ansprüche 1-6, wobei bei dem Individuum
(a) eine chronische Nierenkrankheit vor dem Auftreten von Anämie vorliegt; oder
(b) eine chronische Nierenkrankheit nach dem Auftreten von Anämie vorliegt.

8. Rekombinantes IL-12 zur Verwendung nach einem der Ansprüche 1-6, wobei bei dem Individuum Anämie und Herz-Kreislauf-Erkrankung vorliegen.

## Revendications

1. IL-12 recombinante pour utilisation dans l'augmentation du niveau d'érythropoïétine endogène dans le sang d'un sujet souffrant d'insuffisance rénale chronique, de maladie rénale chronique, de maladie cardiovasculaire ou d'insuffisance cardiaque congestive.

2. IL-12 recombinante pour utilisation selon la revendication 1, où le sujet est humain.

3. IL-12 recombinante pour utilisation selon la revendication 2, où l'IL-12 recombinante est administrée au sujet à une dose :
(a) comprise entre environ 5 ng/kg de poids corporel et environ 500 ng/kg de poids corporel ;
(b) comprise entre environ 10 ng/kg de poids corporel et environ 320 ng/kg de poids corporel ;
(c) comprise entre environ 16 ng/kg de poids corporel et environ 160 ng/kg de poids corporel ; ou
(d) comprise entre environ 5 µg et 15 µg.

4. IL-12 recombinante pour utilisation selon l'une quelconque des revendications 1 à 3, où l'IL-12 recombinante est administrée au sujet en dose unique.

5. IL-12 recombinante pour utilisation selon l'une quelconque des revendications 1 à 3, où l'IL-12 recombinante est administrée au sujet en doses multiples.

6. IL-12 recombinante pour utilisation selon l'une quelconque des revendications 1 à 3 et 5, où l'IL-12 recombinante est :
(a) administrée au sujet une fois par jour, une fois tous les deux jours, une fois tous les 3 jours, une fois tous les 4 jours, une fois tous les 5 jours, une fois tous les 6 jours, une fois par semaine, une fois toutes les deux semaines, une fois toutes les 3 semaines ou une fois toutes les 4 semaines ; ou
(b) administrée au sujet une fois par mois.

7. IL-12 recombinante pour utilisation selon l'une quelconque des revendications 1 à 6, où le sujet souffre :
(a) de maladie rénale chronique avant l'apparition d'une anémie ; ou
(b) de maladie rénale chronique après l'apparition d'une anémie.

8. IL-12 recombinante pour utilisation selon l'une quelconque des revendications 1 à 6, où le sujet souffre d'anémie et d'une maladie cardiovasculaire.
